# EUROPÄISCHE PATENTSCHRIFT

(11) **EP 3 886 734 B1**
(45) Veröffentlichungstag und Bekanntmachung des Hinweises auf die Patenterteilung: **23.08.2023**
(21) Anmeldenummer: 19812994.2
(22) Anmeldetag: 29.11.2019
(51) Int. Cl.: A61B 17/16, A61B 17/00

(54) **SCHLÜSSELLOSE WERKZEUGSCHAFTKOPPLUNG**
KEYLESS TOOL SHAFT COUPLING
COUPLAGE SANS CLÉ D'UN MANCHE D'OUTIL

(30) Priorität: 30.11.2018 DE 102018130576
(43) Veröffentlichungstag der Anmeldung: 06.10.2021
(73) Patentinhaber: Aesculap AG, 78532 Tuttlingen (DE)
(72) Erfinder: SCHAZ, Uwe, 78579 Neuhausen (DE); HÖGERLE, Roland-Alois, 78532 Tuttlingen (DE)
(74) Vertreter: Winter, Brandl - Partnerschaft mbB
(86) Internationale Anmeldenummer: PCT/EP2019/083102
(87) Internationale Veröffentlichungsnummer: WO 2020/109557

(56) Entgegenhaltungen:
- EP-A1- 1 790 292
- US-A1- 2016 074 047

## Beschreibung

Die Erfindung betrifft eine schlüssellose Kopplung für einen Werkzeugschaft und ein Handstück eines chirurgischen Instruments mit einem ersten Kopplungsabschnitt, der an einem proximalen Abschnitt des Werkzeugschafts vorgesehen ist, einem zweiten Kopplungsabschnitt, der an einem distalen Abschnitt einer Schaftaufnahme des Handstücks vorgesehen ist, und einem Ver-/Entriegelungselement.

### Hintergrund der Erfindung

Bei chirurgischen Instrumenten, insbesondere Sägen wie beispielsweise Transversalsägen, können Werkzeugschäfte, sogenannte Werkzeugschäfte, die im Vergleich zum Handstück (Antriebshandstück) des Instruments einen schlanken Durchmesser aufweisen, mit unterschiedlichen Längen verwendet werden, um dem Chirurgen unter anderem durch Auswahl der geeigneten Werkzeugschaftlänge einen guten Zugang zur Operationsstelle zur ermöglichen. Vor dem chirurgischen Einsatz wird der ausgewählte Werkzeugschaft mit dem Handstück mechanisch gekoppelt und nach Beendigung des chirurgischen Einsatzes wieder von diesem gelöst, um den Werkzeugschaft zu reinigen.

Dabei kann das mechanische Verbinden und Lösen der beiden Kopplungsabschnitte am Werkzeugschaft und am Handstück entweder unter Zuhilfenahme eines vorgesehenen Verbindungswerkzeugs bzw. Schlüssels oder werkzeuglos in Form einer sogenannten Schnellkopplung erfolgen.

### Stand der Technik

Aus dem Stand der Technik sind Werkzeugschaftkopplungen / Ver-/Entriegelungsmechanismen bekannt, bei denen die Kopplung des Kopplungsabschnitts am Werkzeugschaft mit dem Kopplungsabschnitt am Handstück durch Einsatz eines speziell angepassten oder standardisierten Kopplungswerkszeugs bzw. Schlüssels erfolgt. Beispielsweise wird der Werkzeugschaft durch Anziehen einer Schraube oder Klemmhülse kraft- und/oder formschlüssig mit dem Handstück gekoppelt.

Verbindungen mit Hilfe eines vorgesehenen Werkzeugs haben jedoch immer den Nachteil, dass der Anwender beim Koppeln des Instruments einen vergleichsweise hohen Aufwand betreiben muss, wenn er ein weiteres zusätzliches Teil, nämlich das Kopplungswerkzeug, handhaben muss. Weiter besteht während des Aufbereitungskreislaufs das Risiko, dass das Kopplungswerkzeug beispielsweise bei der Sterilisation verloren geht oder einem falschen Sieb zugeordnet wird.

Alternative Lösungen aus dem Stand der Technik sehen deshalb eine werkzeuglose bzw. schlüssellose Verbindung der beiden Kopplungsabschnitte vor, beispielsweise in Form einer formschlüssigen Verbindung mittels eines Ver-/Entriegelungselements am Handstück, unter anderem in Form eines Druckknopfes oder einer drehbaren Hülse.

Kopplungen bzw. Ver-/Entriegelungsmechanismen mittels eines am Handstück angeordneten Ver-/Entriegelungselements sind jedoch technisch aufwändig, da meistens das Ver-/Entriegelungselement von der sich bewegenden /beweglichen Schaftaufnahme entkoppelt werden muss. Überdies besteht bei einem Ver-/Entriegelungselement, das am Handstück angeordnet ist, die Gefahr einer unbeabsichtigten Entriegelung während der chirurgischen Anwendung, da der Chirurg das Handstück während der Anwendung meist im Ver-/Entriegelungsbereich festhält und führt. Dadurch entsteht ein erhebliches Risiko für den Patienten.

Dokument EP1790292 A1 offenbart eine Kopplung gemäß der Präambel von Anspruch 1.

### Kurze Beschreibung der Erfindung

Es ist daher Aufgabe der Erfindung, die Nachteile aus dem Stand der Technik zu beseitigen oder zumindest zu mildern. Insbesondere soll eine Werkzeugschaftkopplung für ein chirurgisches Instrument geschaffen werden, dass dem Anwender in einfacher technischer Weise eine komfortable Handhabung des Ver-/Entriegelungsmechanismus ermöglicht und eine Gefahr für den Patienten minimiert.

Die Aufgabe wird gelöst durch eine schlüssellose Werkzeugschaftkopplung für ein (bzw. eines) chirurgisches(n) Instrument(s) bzw. schlüssellose Kopplung für einen (bzw. eines) Werkzeugschaft(s) und ein (bzw. für ein) Handstück eines chirurgischen Instruments mit den Merkmalen des Anspruchs 1. Vorteilhafte Weiterbildungen der Erfindung sind Gegenstand der beigefügten Unteransprüche.

Ein Grundgedanke der Erfindung besteht darin, eine werkzeuglos/schlüssellos handhabbare Werkzeugschaftkopplung für ein chirurgisches Instrument mit einem Ver-/Entriegelungselement bereitzustellen, welches außerhalb des (definierten) Haltebereichs des Handstücks angeordnet ist, genauer gesagt am Werkzeugschaft selbst angeordnet ist.

Konkret wird eine schlüssellose Kopplung bzw. ein schlüsselloser Kopplungsmechanismus oder Ver-/Entriegelungsmechanismus für einen Werkzeugschaft und ein Handstück eines chirurgischen Instruments, insbesondere chirurgische (Transversal)Säge, bereitgestellt mit einem ersten Kopplungsabschnitt, der an einem proximalen (End-)Abschnitt, vorzugsweise in einer proximalen Hälfte, des Werkzeugschafts vorgesehenen ist, einem zweiten Kopplungsabschnitt, der an einem distalen (End-)Abschnitt einer Schaftaufnahme des Handstücks vorgesehen ist, und einem Ver-/Entriegelungselement. Dabei ist das Ver-/Entriegelungselement am Werkzeugschaft (als fester Bestandteil (von diesem)) angeordnet und bildet einen Rastabschnitt aus. Die Schaftaufnahme weist zumindest eine Hinterschneidung, insbesondere Ausnehmung oder (alternativ) Ausbruch / leistenförmiger Vorsprung, beispielsweise Durchgangsöffnung oder Fenster, auf, die dazu vorgesehen und angepasst ist, den Rastabschnitt in einer Verriegelungsposition der beiden Kopplungsabschnitte so aufzunehmen, dass der Rastabschnitt und die Hinterschneidung sich in einem Rasteingriff miteinander befinden und die beiden Kopplungsabschnitte (und folglich der Werkzeugschaft und die Schaftaufnahme bzw. das Handstück) gegen eine relative axiale Bewegung zueinander zumindest in Zugrichtung, also voneinander weg, sichern.

Unter "proximal" wird stets das zum Anwender des chirurgischen Instruments hin gerichtete Ende verstanden, unter "distal" stets das vom Anwender weg gerichtete Ende.

Dadurch, dass das Ver-/Entriegelungselement sich somit außerhalb des (definierten) Haltebereichs am Handstück bzw. grundsätzlich außerhalb des Handstück befindet, ist eine unbeabsichtigte Lösung der Verrastung durch den Benutzer während des Gebrauchs des chirurgischen Instruments nahezu ausgeschlossen. Der Chirurg kann das Gerät folglich komfortabel am Handstück festhalten und führen, ohne dass ein Risiko für den Patienten durch unbeabsichtigtes Ablösen des Werkzeugschafts entsteht.

Der Rastabschnitt kann insbesondere eine in Richtung zum proximalen Ende des Werkzeugschafts schräg verlaufende Abgleitfläche aufweisen. Beim Einschieben des Werkzeugschafts in die Schaftaufnahme kann das Ver-/Entriegelungselement folglich selbstständig in die Schaftaufnahme (elastisch ausweichend) eintauchen, indem der Rastabschnitt an seiner schrägen Abgleitfläche am distalen Ende der Schaftaufnahme entlang gleitet und schließlich in die Hinterschneidung einschnappt ("plug and play" Kupplungsvorgang).

Im Rasteingriff sichern der Rastabschnitt und die Hinterschneidung die beiden Kopplungsabschnitte bevorzugt auch gegen eine relative Bewegung der Kopplungsabschnitte in Umfangsrichtung zueinander. Auf diese Weise sind die beiden Kopplungsabschnitte verdrehfest zueinander positioniert, sodass zum einen die Kopplung stabiler ist und zum anderen ein Drehmoment von der Schaftaufnahme auf den Werkzeugschaft übertragen werden kann.

Das Vorspannelement ist eine Biegefeder, insbesondere eine Nadelfeder, ist, die an ihrem einen Ende um dieselbe Schwenkachse wie das Ver-/Entriegelungselement drehbar in der Nut und in radialer Richtung des Werkzeugschafts unterhalb des Ver-/Entriegelungselements angeordnet ist.

Vorzugsweise weist der Werkzeugschaft eine innenliegende Führungstasche auf, die sich in proximaler Richtung des Werkzeugschafts an die Nut anschließt und die dazu vorgesehen und angepasst ist, das andere Ende der Biegefeder aufzunehmen und einen Schwenkradius der Biegefeder zu begrenzen, insbesondere eine Schwenkbewegung der Biegefeder zu verhindern.

In ein vorteilhaften Ausführungsform sind die Biegefeder und das Ver-/Entriegelungselements um einen Querstift drehbar in der Nut gelagert sind, sodass die Biegefeder am Querstift anliegt und dessen Umfangsseite nahezu vollständig umschließt, zumindest aber mehr als 180°, und bevorzugt 270° umschließt.

Bevorzugt weist das Ver-/Entriegelungselement an seiner Unterseite in radialer Richtung des Werkzeugschafts eine Ausdehnung auf, so dass die Ausdehnung im eingekoppelten Zustand von oben auf die Biegefeder drückt, sodass diese in der Verriegelungsposition entgegen ihrer Ruhelage elastisch verformt wird und das Ver-/Entriegelungselement in eine Richtung radial nach außen des Werkzeugschafts vorspannt.

Weiter vorteilhaft weist der Nutengrund im Bereich des Querstifts eine Ausrundung mit verminderter Wandstärke auf, um bei gleichbleibendem Außendurchmesser des Werkzeugschafts ausreichend Bauraum für den Querstift und die daran anliegende Biegefeder sowie das Ver-/Entriegelungselement bereitzustellen.

Weiter vorteilhaft beträgt der Schwenkradius des Ver-/Entriegelungselements im unverriegelten Zustand der Kopplungsabschnitte mindestens 45°, vorzugsweise mindestens 90°, besonders bevorzugt 180° beträgt.

Vorzugsweise weist der Rastabschnitt eine in Richtung zum proximalen Ende des Werkzeugschafts hin schräg verlaufende Abgleitfläche auf.

Vorzugsweise weist die Schaftaufnahme eine Mehrzahl, bevorzugt zwei, drei oder vier, an in Umfangsrichtung der Schaftaufnahme beabstandeten, insbesondere gleichmäßig beabstandeten, Hinterschneidungen/Ausnehmungen auf, die jeweils dazu vorgesehen und angepasst sind, den Rastabschnitt in der Verriegelungsposition derart aufzunehmen, dass diese sich mit dem Rastabschnitt in Rasteingriff befinden. Vorteilhafterweise kann somit eine weitere Stabilisierung der Kopplung bewirkt werden.

Das Ver-/Entriegelungselement weist insbesondere einen Betätigungsabschnitt auf, der dazu vorgesehen und angepasst ist, durch manuelle Betätigung durch den Anwender den Rastabschnitt aus dem Rasteingriff mit der Hinterschneidung wieder zu lösen und eine relative axiale Verschiebung der beiden Kopplungsabschnitte zueinander zumindest in Zugrichtung wieder freizugeben. Somit wird in vorteilhafterweise eine besonders einfache und komfortable Handhabung des chirurgischen Geräts bei der Entkopplung ermöglicht, die nicht dem Risiko einer unbeabsichtigten Entkopplung während des Geräteeinsatzes am Patienten unterliegt, da der Betätigungsabschnitt nicht am Handstück bzw. im Haltebereich des Handstücks angeordnet ist, sondern am Werkzeugschaft.

Bevorzugt ist zumindest der Rastabschnitt, insbesondere bevorzugt das gesamte Ver-/Entriegelungselement, mittels eines Vorspannelements in eine Richtung radial nach außen des Werkzeugschafts vorgespannt. Auf diese Weise kann mit einfachen Mitteln bewirkt werden, dass der Rastabschnitt in der Verriegelungsposition in der Hinterschneidung gehalten wird und der Rasteingriff folglich verlässlich bestehen bleibt, bis die Vorspannkraft gezielt (manuell) überwunden wird.

Der Werkzeugschaft weist insbesondere eine sich im Wesentlichen in seiner Längsrichtung erstreckende Nut auf, die dazu vorgesehen und angepasst ist, das Ver-/Entriegelungselement in seiner gesamten Länge darin aufzunehmen. Das Ver-/Entriegelungselement wird also baulich mit einem Teil seiner Höhenerstreckung in den Werkzeugschaft versenkt und ermöglicht eine kompakte Bauweise des Werkzeugschaft-Ver-/Entriegelungselement-Einheit.

Vorzugsweise ist das Ver-/Entriegelungselement an seinem distalen Ende um eine Schwenkachse, die quer zur Längsachse des Werkzeugschafts und der Nut verläuft, drehbar in der Nut angeordnet / gelagert, sodass das Ver-/Entriegelungselement aus der Nut heraus und in diese hinein verschwenkt werden kann. Die Schwenkbewegung ermöglicht, dass der Rastabschnitt in die Hinterschneidung an der Schaftaufnahme, also in seine Raststellung, einschwenken und aus dieser wieder ausgeschwenkt werden kann.

Bevorzugt ist das Vorspannelement eine Biegefeder, beispielsweise eine Blattfeder oder Stabfeder mit schmaler Breite, insbesondere schmaler als die Nutbreite, oder besonders bevorzugt eine Draht- oder Nadelfeder, die an ihrem einen (distalen) Ende um dieselbe oder an derselben Schwenkachse wie das Ver-/Entriegelungselement drehbar oder drehfest in der Nut (entlang ihrer Längserstreckung) angeordnet bzw. gelagert oder festgelegt ist und sich in radialer Richtung des Werkzeugschafts unterhalb des Ver-/Entriegelungselement befindet. Diese Konstruktion ermöglicht vorteilhafterweise eine besonders kompakte Bauweise des Werkzeugschafts mit Verriegelungselement.

Weiter kann der Werkzeugschaft vorzugsweise eine innenliegende / in das Innere des Werkzeugschafts eingebrachte Führungstasche aufweisen, die sich in proximaler Richtung des Werkzeugschafts an die Nut anschließt und die dazu vorgesehen und angepasst ist, das andere (proximale) Ende der Biegefeder aufzunehmen und insbesondere einen Schwenkradius der Biegefeder zu begrenzen oder eine Schwenkbewegung der Biegefeder ganz zu verhindern. Dies ermöglicht zum einen eine stabile Positionierung der Biegefeder und verhindert zum anderen, dass die Biegefeder in unverriegeltem Zustand der Kupplungsabschnitte aus der Nut des Werkzeugschafts heraus schwenken kann und eine Verletzungsgefahr für den Anwender darstellt.

Insbesondere beträgt der Schwenkradius des Ver-/Entriegelungselements im unverriegelten Zustand der Kopplungsabschnitte mindestens 45°, vorzugsweise mindestens 90°, besonders bevorzugt 180°. Im unverriegelten Zustand kann das Ver-/Entriegelungselement somit im Rahmen seines vorgegebenen Schwenkradius frei aus der Nut heraus schwenken und die Nut mitsamt innenliegender Biegefeder und Führungstasche für ein Reinigungsmittel freigeben. Je größer dabei der vorgegebene Schwenkradius ist, desto einfach sind Nut, Führungstasche und Biegefeder für die Reinigung zugänglich.

### Kurzbeschreibung der Figuren

Die Erfindung wird nachstehend anhand eines bevorzugten Ausführungsbeispiels unter Bezugnahme auf die beigefügte Zeichnung näher beschrieben. Es zeigen:
Fig. 1 eine perspektivische Draufsicht eines Werkzeugschafts und einer Schaftaufnahme in ungekoppeltem Zustand gemäß einer Ausführungsform der Erfindung;
Fig. 2 eine perspektivische Draufsicht des Werkzeugschafts und der Schaftaufnahme in gekoppeltem Zustand gemäß der Ausführungsform der Erfindung;
Fig. 3 eine perspektivische Draufsicht eines chirurgischen Instruments mit eingekoppeltem Werkzeugschaft gemäß der Ausführungsform der Erfindung;
Fig. 4 einen Ausschnitt eines Längsschnitts durch den Werkzeugschaft und die Schaftaufnahme in gekoppeltem Zustand entlang der Linie IV in Fig. 2;
Fig. 5 eine perspektivische Seitenansicht des Werkzeugschafts in einer Reinigungsposition;
Fig. 6 eine perspektivische Unteransicht des Werkzeugschafts in der Reinigungsposition;
Fig. 7 eine perspektivische Seitenansicht des Werkzeugschafts in einer Reinigungshalterung.

Die Figuren sind lediglich schematischer Natur und dienen ausschließlich dem Verständnis der Erfindung. Die gleichen Elemente sind mit denselben Bezugszeichen bezeichnet.

### Detaillierte Beschreibung bevorzugter Ausführungsbeispiele

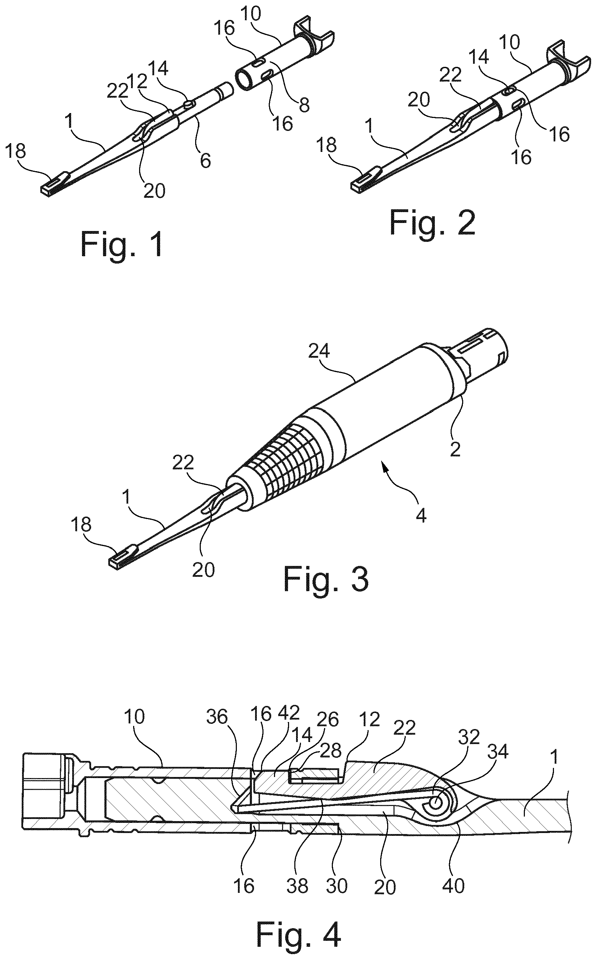

Fig. 1 zeigt in perspektivischer Draufsicht eine schlüssellos bedienbare Kopplung für einen (bzw. eines) Werkzeugschaft(s) 1 und eine Schaftaufnahme 10, die Teil eines Handstücks 2 (in Fig. 3 gezeigt) für ein (bzw. eines) chirurgisches(n) Instrument(s) 4 ist, mit einem ersten Kopplungsabschnitt 6, der in einem proximalen Abschnitt des Werkzeugschafts 1 vorgesehenen ist, einem zweiten Kopplungsabschnitt 8, der in einem distalen Abschnitt der Schaftaufnahme 10 vorgesehen ist, und einem Ver-/Entriegelungselement 12, das am Werkzeugschaft 1 (nicht entfernbar) angeordnet ist und einen Rastabschnitt 14 ausbildet. Die (weibliche) Schaftaufnahme 10 weist zumindest eine (senkrecht zur Schaftaufnahmerichtung ausgerichtete) Ausnehmung 16 auf, die dazu vorgesehen und angepasst ist, den Rastabschnitt 14 in einer Verriegelungsposition (in Fig. 2 gezeigt) der beiden Kopplungsabschnitte 6, 8 aufzunehmen, sodass der Rastabschnitt 14 und die Ausnehmung 16 sich in Rasteingriff zueinander befinden und die beiden Kopplungsabschnitte 6, 8 gegen eine relative axiale Bewegung zueinander zumindest in Zugrichtung (Schaftaufnahmerichtung) sichern.

Fig. 1 zeigt den Werkzeugschaft 1 und die Schaftaufnahme 10 in ungekoppeltem Zustand gemäß einer Ausführungsform der Erfindung. Am distalen Ende des Werkzeugschafts 1 ist eine Effektoraufnahme 18, beispielsweise zur Aufnahme eines separaten Sägeblatts (nicht gezeigt), vorgesehen. Der abgebildete Werkzeugschaft 1 ist ausgehend von der Effektoraufnahme 18 bis in etwa seiner halben Längserstreckung flach ausgebildet und nimmt in Richtung des proximalen Endes in seiner Breite und Höhe/Dicke (orthogonal zur Längserstreckung) zu. An diesen flachen Teil des Werkzeugschafts 1 schließt sich mit gerundetem Übergang ein Werkzeugschaftabschnitt mit (im Wesentlichen) rundem Querschnitt an, der im Bereich seines proximalen Endes eine umlaufende Ringnut aufweist. Die Ringnut dient beispielsweise dazu, den Werkzeugschaft zur Reinigung/Desinfektion/Sterilisation in einer Reinigungshalterung zu halten, wozu beispielsweise an der Reinigungshalterung Fixierungselemente, insbesondere federnde Klemmbleche, vorgesehen sind, die in die Ringnut eingreifen und den Schaft axial, vorzugsweise axial und radial fixieren. Am proximalen Ende des Werkzeugschafts 1 ist weiter eine Fase vorgesehen.

Ausgehend von dem gerundeten Übergang am flachen Teil zu dem Werkzeugschaftabschnitt runden Querschnitts ist in Richtung des proximalen Endes des Werkzeugschafts 1 eine längliche Nut 20 in den Werkzeugschaft 1 eingebracht, die sich entlang der Längsachse des Werkzeugschafts 1 erstreckt und deren Breite und Länge auf die Breite und Länge des Ver-/Entriegelungselements 12 abgestimmt sind. In der Nut 20 ist das Ver-/Entriegelungselement 12 bis zu einem Teil seiner Höhe aufgenommen. Dabei weist das Ver-/Entriegelungselement 12 an seinem proximalen Ende einen Rastabschnitt 14 in Form eines Rasthakens und an seinem distalen Ende einen Betätigungsabschnitt 22 auf, die im ungekoppelten Zustand des Werkzeugschafts 1 beide über die Oberfläche des Werkzeugschafts 1 hinausragen. Der Längsabschnitt des Werkzeugschafts 1, in dem sich der Rastabschnitt 14 des Ver-/Entriegelungselements 12 befindet bis zum proximalen Ende des Werkzeugschafts 1, stellt den ersten Kupplungsabschnitt 6 des Werkzeugschafts 1 dar.

Die Schaftaufnahme 10, welche fest im Handstück 2 eines chirurgischen Instruments 4 (siehe Fig. 3) verbaut ist, weist einen distalen Bereich in Form eines Hohlzylinders auf, dessen Innendurchmesser auf den Außendurchmesser des ersten Kupplungsabschnitts 6 abgestimmt ist, um diesen aufzunehmen. An den Hohlzylinder schließt sich in proximaler Richtung ein verbreiteter, in etwa halbmondförmiger Abschnitt an, an den der Hohlzylinder ungefähr mittig angesetzt oder einstückig angeformt ist. Im distalen Bereich des Hohlzylinders sind vier Ausnehmungen 16 in Form von Durchgangsöffnungen bzw. Fenstern eingebracht, die in Umgangsrichtung des Hohlzylinders gleichmäßig beabstandet sind. Die Größe der Ausnehmungen 16 ist so gewählt, dass der Rastabschnitt 14 darin aufgenommen werden kann. Der Längsabschnitt des Hohlzylinders, in den der erste Kopplungsabschnitt 6 des Werkzeugschafts 1 eingeschoben werden kann, bis der Rastabschnitt 14 in eine der Ausnehmungen 16 einschnappt, bildet den zweiten Kopplungsabschnitt 8 der Schaftaufnahme 10.

In Fig. 2 ist eine perspektivische Draufsicht des Werkzeugschafts 1 und der Schaftaufnahme 10 in gekoppeltem Zustand gemäß der Ausführungsform der Erfindung dargestellt. In gekoppeltem Zustand ist der Rastabschnitt 14 in eine der Ausnehmungen 16 eingerastet und sichert die Kopplungsabschnitte 6, 8 gegen eine axiale Zugbewegung zueinander. Auch eine relative Drehbewegung der Kopplungsabschnitte 6, 8 ist in verrastetem Zustand des Rastabschnitts 14 in der Ausnehmung 16 verhindert, da die längsverlaufenden Flächen des Rastabschnitts 14 mit den längsverlaufenden Wänden der Ausnehmung 16 in Anlage treten und eine Drehbewegung formschlüssig blockieren. Je nach gewünschter Winkelstellung der Effektoraufnahme 18 zur Schaftaufnahme 10 bzw. zum Handstück 2 kann eine der vier Ausnehmungen 16 als Rastausnehmung für den Rastabschnitt 14 gewählt werden.

In Fig. 3 ist eine perspektivische Draufsicht eines chirurgischen Instruments 4 mit eingekoppeltem Werkzeugschaft 1 gemäß der Ausführungsform der Erfindung gezeigt. Zu sehen ist das Handstück 2 des chirurgischen Instruments 4 mit einem Griffbereich 24 zum Fassen und Führen des chirurgischen Instruments 4 durch den Anwender. Die Schaftaufnahme 10 ist im Inneren des Handstücks 2 verbaut und deshalb in der Fig. 3 nicht zu sehen. Aus dem distalen Ende des Handstücks 2 ragt der eingekoppelte Werkzeugschaft 1 soweit heraus, dass der Betätigungsabschnitt 22 des Ver-/Entriegelungselements 12 sich unmittelbar distal zum Handstück 2, also außerhalb des Handstücks 2, befindet.

Fig. 4 stellt einen Ausschnitt eines Längsschnitts durch den Werkzeugschaft 1 und die Schaftaufnahme 10 in gekoppeltem Zustand entlang der Linie IV der Fig. 2 dar. Zu sehen ist, dass der Rastabschnitt 14 in eine Ausnehmung 16 eingerastet ist. Dabei tritt der Rastabschnitt 14 mit einer Rastfläche 26, die sich im Wesentlichen senkrecht zur Längserstreckung des Werkzeugschafts 1 radial nach außen erhebt, mit einer Kontaktfläche 28 der Ausnehmung 16, die der Rastfläche 26 in gekoppeltem Zustand gegenüber liegt, in Anlage und blockiert somit durch Formschluss eine Zugbewegung (relative axiale Bewegung voneinander weg) der beiden Kopplungsabschnitte 6, 8. Zu sehen ist weiter, dass der Werkzeugschaft 1 an seiner dem Betätigungselement 22 radial gegenüberliegenden Seite eine radiale Stufe oder Schulter 30 ausbildet, deren Stirnseite (der Schaftaufnahme 10 zugewandte Seite) mit einer distalen Endfläche (dem Werkzeugschaft 1 zugewandte Seite) der Schaftaufnahme 10 in Anlage tritt und somit eine Schubbewegung (relative axiale Bewegung aufeinander zu) der beiden Kopplungsabschnitte 6, 8 formschlüssig blockiert. Das Betätigungselement 22 selbst nimmt im eingekoppelten Zustand eine Höhe (Erstreckung in radialer Richtung des Werkzeugschafts 1) ein, die die Oberfläche/Außenfläche der Schaftaufnahme 10 überschreitet, während die Höhe des Rastabschnitts 14 so gewählt ist, dass dieser im eingekoppelten Zustand in etwa bündig mit der Oberfläche der Schaftaufnahme 10 liegt. Der Längsabstand zwischen dem Rastabschnitt 14 und dem Betätigungselement 22 des Ver-/Entriegelungselements 12 überschreitet die Längsausdehnung der Schaftaufnahme 10 zwischen ihrem distalen Ende und der Ausnehmung 16 dabei minimal, sodass der Abschnitt der Schaftaufnahme 10 zwischen ihrem distalen Ende und der Ausnehmung 16 im eingekoppelten Zustand zwischen dem Rastabschnitt 14 und dem Betätigungselement 22 aufgenommen ist.

Unterhalb (radial innen) des Ver-/Entriegelungselements 12 ist eine Biegefeder 32 angeordnet. Die Biegefeder 32 ist in dieser Ausführungsform ein Draht. Sowohl die Biegefeder 32 als auch das Ver-/Entriegelungselements 12 sind um einen Querstift 34 drehbar in der Nut 20 angeordnet bzw. gelagert. Der Querstift 34 bildet somit eine Schwenkachse für das Ver-/Entriegelungselement 12 und die Biegefeder 32 und ist am distalen Ende bzw. Abschnitt der Nut 20 vorgesehen. Dabei liegt die Biegefeder 32 am Querstift 34 an und umschließt dessen Umfangsseite nahezu vollständig, zumindest aber mehr als 180°, und bevorzugt 270°. Ausgehend von der den Querstift 34 umgebenden Biegung erstreckt sich die Biegefeder 32 in Längsrichtung der Nut 20 bis in eine Führungstasche 36 hinein, die sich proximal an die Nut 20 anschließt und in das Innere des Werkzeugschafts 1 eingebracht ist, also keine Öffnung zur Umfangsseite des Werkzeugschafts 1 hin hat. Die Führungstasche 36 hat in radialer Richtung zum Ver-/Entriegelungselement 12 hin eine abgeschrägte Wandfläche, die den Schwenkradius der Biegefeder 32 in radialer Richtung begrenzt, sodass diese nicht aus der Führungstasche 36 hinaus schwenken kann.

Das Ver-/Entriegelungselement 12 wiederum liegt an seinem distalen Ende an der den Querstift 34 umgebenden Biegung der Biegefeder 32 an und umschließt deren Umfangsseite vorzugsweise um mehr als 180°. An seiner Unterseite zeigt das Ver-/Entriegelungselement 12 in radialer Richtung des Werkzeugschafts 1 eine Ausdehnung 38, die in der Fig. 4 als leichtes V-Profil zu erkennen ist. Im eingekoppelten Zustand drückt die Ausdehnung 38 von oben auf die Biegefeder 32, sodass diese entgegen ihrer Ruhelage elastisch verformt wird und das Ver-/Entriegelungselement 12 in eine Richtung radial nach außen des Werkzeugschafts 1 vorspannt. Auf diese Weise sichert die Biegefeder 32 den Rasteingriff des Rastabschnitts 14 in der Ausnehmung 16. Im Bereich des Querstifts 34 hat der Nutengrund eine Ausrundung 40 mit verminderter Wandstärke, um bei gleichbleibendem Außendurchmesser des Werkzeugschafts 1 ausreichend Bauraum für den Querstift 34 und die daran anliegende Biegefeder 32 sowie das Ver-/Entriegelungselement 12 zu schaffen.

Der Querstift 34 selbst ist in einer Durchgangsbohrung (nicht gezeigt) der Nutwände gehalten und schließt bündig mit der Oberfläche des Werkzeugschafts 1 ab. Gesichert wird der Querstift 34 durch eine Presspassung oder durch Verschweißen.

Sollen der Werkzeugschaft 1 und die Schaftaufnahme 10 miteinander gekoppelt werden, so werden die beiden Kopplungsabschnitte 6, 8 manuell ineinander geschoben. Dabei gleitet der Rastabschnitts 14 an seiner schrägen Abgleitfläche 42 ohne manuelle Betätigung des Ver/Entriegelungselements 12 am distalen Ende des zweiten Kupplungsabschnitts 8 entlang und wird entgegen der Federkraft der Biegefeder 32 radial nach innen gedrückt, bis er den Längsabschnitt der Schaftaufnahme 10 zwischen ihrem distalen Ende und der Ausnehmung 16 passiert hat und durch die Vorspannkraft der Biegefeder 32 in die Ausnehmung 16 einschnappt.

Sollen der Werkzeugschaft 1 und die Schaftaufnahme voneinander entkoppelt werden, so wird der Betätigungsabschnitt 22 entgegen der Vorspannkraft der Biegefeder 32 manuell nach innen gedrückt, wodurch auch der Rastabschnitt 14 radial nach innen und aus der Ausnehmung 16 heraus bewegt wird, sodass der Rasteingriff von Rastabschnitt 14 und Ausnehmung 16 aufgehoben wird und der Werkzeugschaft 1 manuell aus der Schaftaufnahme 10 herausgezogen werden kann.

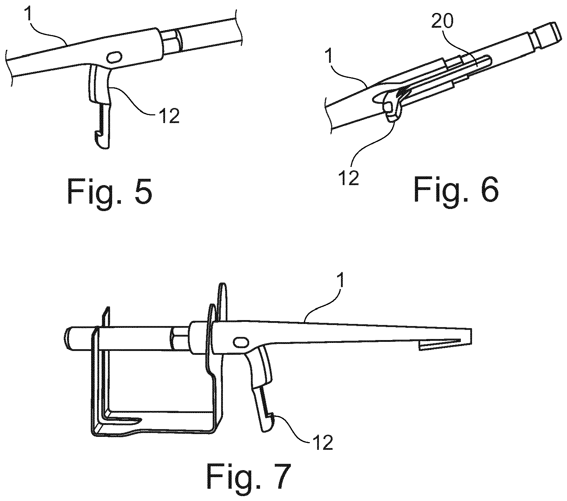

In Fig. 5 ist eine perspektivische Seitenansicht des Werkzeugschafts 1 in einer Reinigungsposition gezeigt. Ist der Werkzeugschaft 1 entkoppelt, kann er durch Drehen des Ver-/Entriegelungselements 12 zur Unterseite des Werkzeugschafts 1 in seine Reinigungsposition gebracht werden. Schwerkraftbedingt schwenkt das Ver-/Entriegelungselements 12 aus der Nut 20 heraus nach unten und gibt somit die Nut 20 und den Zugang zu Biegefeder 32 und Führungstasche 36 für ein Reinigungsmittel und/oder -instrument frei. Dadurch, dass die Führungstasche 36 den Schwenkradius der Biegefeder 32 begrenzt, verlässt diese die Nut 20 nicht, sondern wird auch in der Reinigungsposition in der Nut 20 und der Führungstasche 36 gehalten, was eine Verletzungsgefahr des Anwenders durch die Biegefeder 36 ausräumt.

Fig. 6 zeigt eine perspektivische Unteransicht des Werkzeugschafts 1 in der Reinigungsposition. In dieser Darstellung ist zu erkennen, wie die Nut 20 in der Reinigungsposition freigelegt ist.

Fig. 7 zeigt eine perspektivische Seitenansicht des Werkzeugschafts 1 in einer Reinigungshalterung. Die Reinigungshalterung lagert den proximalen Abschnitt des Werkzeugschafts 1 im Wesentlichen waagrecht in einer Höhe, in der das Ver-/Entriegelungselement 12 möglichst weit, vorzugweise bis zu einer senkrechten Ausschwenkung, aus der Nut ausschwenken kann. Das Ver-/Entriegelungselement 12 ist folglich automatisch ausklappbar zur maschinellen Reinigung.

Zusammenfassend betrifft die Erfindung eine schlüssellose Kopplung für einen Werkzeugschaft 1 und ein Handstück 2 eines chirurgischen Instruments 4 mit einem ersten Kopplungsabschnitt 6, der an einem proximalen Abschnitt des Werkzeugschafts 1 vorgesehenen ist, einem zweiten Kopplungsabschnitt 8, der an einem distalen Abschnitt einer Schaftaufnahme 10 des Handstücks 2 vorgesehen ist, und einem Ver-/Entriegelungselement 12, wobei das Ver-/Entriegelungselement 12 am Werkzeugschaft 1 angeordnet ist und einen Rastabschnitt 14 ausbildet und die Schaftaufnahme 10 zumindest eine Hinterschneidung, insbesondere Ausnehmung 16, aufweist, die dazu vorgesehen und angepasst ist, den Rastabschnitt 14 in einer Verriegelungsposition der beiden Kopplungsabschnitte 6, 8 aufzunehmen, sodass der Rastabschnitt 14 und die Hinterschneidung, insbesondere Ausnehmung 16, sich in Rasteingriff zueinander befinden und die beiden Kopplungsabschnitte 6, 8 gegen eine relative axiale Bewegung zueinander zumindest in Zugrichtung sichern.

### Bezugszeichenliste

- 1: Werkzeugschaft
- 2: Handstück
- 4: Chirurgisches Instrument
- 6: Erster Kopplungsabschnitt
- 8: Zweiter Kopplungsabschnitt
- 10: Schaftaufnahme
- 12: Ver-/Entriegelungselement
- 14: Rastabschnitt
- 16: Ausnehmung
- 18: Effektoraufnahme
- 20: Nut
- 22: Betätigungsabschnitt
- 24: Griffbereich
- 26: Rastfläche
- 28: Kontaktfläche
- 30: Schulter
- 32: Biegefeder
- 34: Querstift
- 36: Führungstasche
- 38: Ausdehnung
- 40: Ausrundung
- 42: Abgleitfläche

## Patentansprüche

1. Schlüssellose Kopplung für einen Werkzeugschaft (1) und ein Handstück (2) eines chirurgischen Instruments (4) mit einem ersten Kopplungsabschnitt (6), der an einem proximalen Abschnitt des Werkzeugschafts (1) vorgesehenen ist, einem zweiten Kopplungsabschnitt (8), der an einem distalen Abschnitt einer Schaftaufnahme (10) des Handstücks (2) vorgesehen ist, und einem Ver-/Entriegelungselement (12), wobei
- das Ver-/Entriegelungselement (12) am Werkzeugschaft (1) angeordnet ist und einen Rastabschnitt (14) ausbildet und die Schaftaufnahme (10) zumindest eine Ausnehmung (16) aufweist, die dazu vorgesehen und angepasst ist, den Rastabschnitt (14) in einer Verriegelungsposition der beiden Kopplungsabschnitte (6, 8) aufzunehmen, sodass der Rastabschnitt (14) und die Ausnehmung (16) sich in Rasteingriff zueinander befinden und die beiden Kopplungsabschnitte (6, 8) gegen eine relative axiale Bewegung zueinander in Zugrichtung und in Umfangsrichtung sichern, wobei
- das Ver-/Entriegelungselement (12) in der Verriegelungsposition mittels eines Vorspannelements (32) in eine Richtung radial nach außen des Werkzeugschafts (1) vorgespannt ist,
- der Werkzeugschaft (1) eine sich im Wesentlichen in seiner Längsrichtung erstreckende Nut (20) aufweist, die dazu vorgesehen und angepasst ist, das Ver-/Entriegelungselement (22) in seiner gesamten Länge aufzunehmen und
- das Ver-/Entriegelungselement (22) an seinem distalen Ende um eine Schwenkachse drehbar in der Nut (20) angeordnet ist, sodass das Ver-/Entriegelungselement (22) aus der Nut (20) heraus und in diese hinein verschwenkbar ist, **dadurch gekennzeichnet, dass** das Vorspannelement (32) eine Biegefeder ist.

2. Kopplung nach Anspruch 1, **dadurch gekennzeichnet, dass** die Biegefeder, insbesondere eine Nadelfeder, ist, die an ihrem einen Ende um dieselbe Schwenkachse wie das Ver-/Entriegelungselement (22) drehbar in der Nut (20) und in radialer Richtung des Werkzeugschafts (1) unterhalb des Ver-/Entriegelungselements (12) angeordnet ist.

3. Kopplung nach einem der Ansprüche 1 oder 2, **dadurch gekennzeichnet, dass** der Werkzeugschaft (1) eine innenliegende Führungstasche (36) aufweist, die sich in proximaler Richtung des Werkzeugschafts (1) an die Nut (20) anschließt und die dazu vorgesehen und angepasst ist, das andere Ende der Biegefeder (32) aufzunehmen und einen Schwenkradius der Biegefeder (32) zu begrenzen, insbesondere eine Schwenkbewegung der Biegefeder (32) zu verhindern.

4. Kopplung nach einem der Ansprüche 1 bis 3, **dadurch gekennzeichnet, dass** die Biegefeder (32) und das Ver-/Entriegelungselements (12) um einen Querstift (34) drehbar in der Nut (20) gelagert sind und dass die Biegefeder (32) am Querstift (34) anliegt und dessen Umfangsseite nahezu vollständig umschließt, zumindest aber mehr als 180°, und bevorzugt 270° umschließt.

5. Kopplung nach Anspruch 4, **dadurch gekennzeichnet, dass** das Ver-/Entriegelungselement (12) an seiner Unterseite in radialer Richtung des Werkzeugschafts (1) eine Ausdehnung (38) aufweist, so dass die Ausdehnung (38) im eingekoppelten Zustand von oben auf die Biegefeder (32) drückt, sodass diese in der Verriegelungsposition entgegen ihrer Ruhelage elastisch verformt wird und das Ver-/Entriegelungselement (12) in eine Richtung radial nach außen des Werkzeugschafts (1) vorspannt.

6. Kopplung nach einem der Ansprüche 4 oder 5, **dadurch gekennzeichnet, dass** der Nutengrund im Bereich des Querstifts (34) eine Ausrundung (40) mit verminderter Wandstärke aufweist, um bei gleichbleibendem Außendurchmesser des Werkzeugschafts (1) ausreichend Bauraum für den Querstift (34) und die daran anliegende Biegefeder (32) sowie das Ver-/Entriegelungselement (12) bereitzustellen.

7. Kupplung nach einem der Ansprüche 1 bis 6, **dadurch gekennzeichnet, dass** der Schwenkradius des Ver-/Entriegelungselements (12) im unverriegelten Zustand der Kopplungsabschnitte (6, 8) mindestens 45°, vorzugsweise mindestens 90°, besonders bevorzugt 180° beträgt.

8. Kopplung nach einem der Ansprüche 1 bis 7, **dadurch gekennzeichnet, dass** der Rastabschnitt (14) eine in Richtung zum proximalen Ende des Werkzeugschafts (1) hin schräg verlaufende Abgleitfläche (42) aufweist.

9. Kopplung nach einem der Ansprüche 1 bis 8, **dadurch gekennzeichnet, dass** die Schaftaufnahme (10) eine Mehrzahl, bevorzugt zwei, drei oder vier, an in Umfangsrichtung der Schaftaufnahme (10) beabstandeten, insbesondere gleichmäßig beabstandeten, Hinterschneidungen aufweist, die jeweils dazu vorgesehen und angepasst sind, den Rastabschnitt (14) in der Verriegelungsposition derart aufzunehmen, dass diese sich mit dem Rastabschnitt (14) in Rasteingriff befinden.

10. Kopplung nach einem der Ansprüche 1 bis 9, **dadurch gekennzeichnet, dass** das Ver-/Entriegelungselement (12) weiter einen Betätigungsabschnitt (22) aufweist, der dazu vorgesehen und angepasst ist, den Rastabschnitt (14) durch manuelle Betätigung aus dem Rasteingriff mit der Hinterschneidung zu lösen und eine relative axiale Verschiebung der beiden Kopplungsabschnitte (6, 8) zueinander zumindest in Zugrichtung freizugeben.

## Claims

1. A keyless coupling for a tool shaft (1) and a handpiece (2) of a surgical instrument (4), comprising a first coupling portion (6) provided at a proximal portion of the tool shaft (1), a second coupling portion (8) provided at a distal portion of a shaft receptacle (10) of the handpiece (2), and a locking/unlocking element (12), **characterised in that**
- the locking/unlocking element (12) is arranged on the tool shaft (1) and forms a latch portion (14) and the shaft receptacle (10) comprises at least one recess (16) provided and adapted to receive the latch portion (14) in a locking position of the two coupling portions (6, 8) so that the latch portion (14) and the recess (16) are in latching engagement with each other and secure the two coupling portions (6, 8) against relative axial movement with respect to each other in the pulling direction and in the circumferential direction, wherein
- the locking/unlocking element (12) is preloaded in the locking position by means of a preloading element (32) in a direction radially outward of the tool shaft (1), wherein the preloading element (32) is a flexible spring, and
- the tool shaft (1) has a groove (20) extending substantially in its longitudinal direction, wherein the groove is provided and adapted to receive the locking/unlocking element (22) along its entire length, and
- that the locking/unlocking element (22) is arranged at its distal end to be rotatable about a pivot axis in the groove (20), so that the locking/unlocking element (22) is pivotable out of and into the groove (20).

2. The coupling according to claim 1, **characterized in that** the flexible spring is in particular a needle spring, which at its one end is arranged to be rotatable about the same pivot axis as the locking/unlocking element (22) in the groove (20) and in the radial direction of the tool shaft (1) below the locking/unlocking element (12).

3. The coupling according to one of the claims 1 or 2, **characterized in that** the tool shaft (1) has a guiding pocket (36) on the inside which adjoins the groove (20) in the proximal direction of the tool shaft (1) and which is provided and adapted to receive the other end of the flexible spring (32) and to limit a pivot radius of the flexible spring (32), in particular to prevent a pivoting movement of the flexible spring (32).

4. The coupling according to one of claims 1 to 3, **characterized in that** the flexible spring (32) and the locking/unlocking element (12) are mounted rotatably about a transverse pin (34) in the groove (20), and **in that** the flexible spring (32) bears against the transverse pin (34) and encloses its circumferential side almost completely, but at least encloses more than 180°, and preferably 270°.

5. The coupling according to claim 4, **characterized in that** the locking/unlocking element (12) has an extension (38) on its lower side in the radial direction of the tool shaft (1), so that the extension (38) presses from above onto the flexible spring (32) in the coupled state, so that the latter is elastically deformed in the locking position against its rest position and pretensions the locking/unlocking element (12) in a direction radially outward of the tool shaft (1).

6. The coupling according to one of claims 4 or 5, **characterized in that** the groove bottom in the region of the transverse pin (34) has a bulge (40) with reduced wall thickness in order to provide sufficient installation space for the transverse pin (34) and the flexible spring (32) resting thereon as well as the locking/unlocking element (12) while the outer diameter of the tool shaft (1) remains constant.

7. The coupling according to one of claims 1 to 6, **characterized in that** the pivot radius of the locking/unlocking element (12) in the unlocked state of the coupling portions (6, 8) is at least 45°, preferably at least 90°, particularly preferably 180°.

8. The coupling according to one of claims 1 to 7, **characterized in that** the latch portion (14) has a sliding surface (42) sloping towards the proximal end of the tool shaft (1).

9. The coupling according to one of claims 1 to 8, **characterized in that** the shaft receptacle (10) has a plurality of, preferably two, three or four, undercuts spaced apart in the circumferential direction of the shaft receptacle (10), in particular evenly spaced apart, which are each provided and adapted to receive the latch portion (14) in the locking position in such a way that they are in latching engagement with the latch portion (14).

10. The coupling according to one of claims 1 to 9, **characterized in that** the locking/unlocking element (12) further comprises an actuation portion (22) provided and adapted to release the latch portion (14) from the latching engagement with the undercut by manual actuation and to release a relative axial displacement of the two coupling portions (6, 8) with respect to each other at least in the pulling direction.

## Revendications

1. Couplage sans clé pour un manche d'outil (1) et une pièce à main (2) d'un instrument chirurgical (4) avec une première section de couplage (6) qui est prévue au niveau d'une section proximale du manche d'outil (1), une seconde section de couplage (8) qui est prévue au niveau d'une section distale d'un logement de manche (10) de la pièce à main (2), et un élément de verrouillage/déverrouillage (12), dans lequel
- l'élément de verrouillage/déverrouillage (12) est agencé au niveau du manche d'outil (1) et réalise une section d'encliquetage (14) et le logement de manche (10) présente au moins un évidement (16) qui est prévu et adapté afin de recevoir la section d'encliquetage (14) dans une position de verrouillage des deux sections de couplage (6, 8) de sorte que la section d'encliquetage (14) et l'évidement (16) se trouvent en prise d'encliquetage l'un avec l'autre et bloquent les deux sections de couplage (6, 8) contre un mouvement axial relatif l'un par rapport à l'autre dans le sens de traction et dans le sens périphérique, dans lequel
- l'élément de verrouillage/déverrouillage (12) est précontraint dans la position de verrouillage au moyen d'un élément de précontrainte (32) dans un sens radialement vers l'extérieur du manche d'outil (1),
- le manche d'outil (1) présente une rainure (20) s'étendant sensiblement dans son sens longitudinal qui est prévue et adaptée afin de recevoir l'élément de verrouillage/déverrouillage (22) dans sa longueur entière et
- l'élément de verrouillage/déverrouillage (22) est agencé à son extrémité distale de manière rotative autour d'un axe de pivotement dans la rainure (20) de sorte que l'élément de verrouillage/déverrouillage (22) puisse être pivoté hors de la rainure (20) et dans celle-ci, **caractérisé en ce que** l'élément de précontrainte (32) est un ressort de flexion.

2. Couplage selon la revendication 1, **caractérisé en ce que** le ressort de flexion est en particulier un ressort d'aiguille qui est agencé à l'une extrémité de manière rotative autour du même axe de pivotement que l'élément de verrouillage/déverrouillage (22) dans la rainure (20) et dans le sens radial du manche d'outil (1) en dessous de l'élément de verrouillage/déverrouillage (12).

3. Couplage selon l'une quelconque des revendications 1 ou 2, **caractérisé en ce que** le manche d'outil (1) présente une poche de guidage (36) intérieure qui se raccorde dans le sens proximal du manche d'outil (1) à la rainure (20) et qui est prévue et adaptée afin de recevoir l'autre extrémité du ressort de flexion (32) et de délimiter un rayon de pivotement du ressort de flexion (32), en particulier d'empêcher un mouvement de pivotement du ressort de flexion (32).

4. Couplage selon l'une quelconque des revendications 1 à 3, **caractérisé en ce que** le ressort de flexion (32) et l'élément de verrouillage/déverrouillage (12) sont logés de manière rotative autour d'une tige transversale (34) dans la rainure (20) et que le ressort de flexion (32) repose contre la tige transversale (34) et entoure presque complètement son côté périphérique, toutefois au moins plus de 180°, et de préférence 270°.

5. Couplage selon la revendication 4, **caractérisé en ce que** l'élément de verrouillage/déverrouillage (12) présente sur son côté inférieur dans le sens radial du manche d'outil (1) une extension (38) de sorte que l'extension (38) presse dans l'état couplé par le haut sur le ressort de flexion (32) de sorte que celui-ci soit élastiquement déformé dans la position de verrouillage dans le sens inverse à sa position de repos et précontraigne l'élément de verrouillage/déverrouillage (12) dans un sens radialement vers l'extérieur du manche d'outil (1).

6. Couplage selon l'une quelconque des revendications 4 ou 5, **caractérisé en ce que** le fond de rainure dans la zone de la tige transversale (34) présente un arrondi (40) avec une épaisseur de paroi réduite afin de fournir, en cas de diamètre extérieur constant du manche d'outil (1), suffisamment d'espace de construction pour la tige transversale (34) et le ressort de flexion (32) reposant dessus ainsi que l'élément de verrouillage/déverrouillage (12).

7. Couplage selon l'une quelconque des revendications 1 à 6, **caractérisé en ce que** le rayon de pivotement de l'élément de verrouillage/déverrouillage (12) s'élève dans l'état déverrouillé des sections de couplage (6, 8) au moins à 45°, de préférence au moins à 90°, le plus préférentiellement à 180°.

8. Couplage selon l'une quelconque des revendications 1 à 7, **caractérisé en ce que** la section d'encliquetage (14) présente une surface de glissement (42) s'étendant en biais en direction de l'extrémité proximale du manche d'outil (1).

9. Couplage selon l'une quelconque des revendications 1 à 8, **caractérisé en ce que** le logement de manche (10) présente une pluralité, de référence deux, trois ou quatre, contre-dépouilles espacées dans le sens périphérique du logement de manche (10), en particulier espacées uniformément qui sont prévues et adaptées respectivement afin de recevoir la section d'encliquetage (14) dans la position de verrouillage de telle manière que celles-ci se trouvent en prise d'encliquetage avec la section d'encliquetage (14).

10. Couplage selon l'une quelconque des revendications 1 à 9, **caractérisé en ce que** l'élément de verrouillage/déverrouillage (12) présente en outre une section d'actionnement (22) qui est prévue et adaptée afin de détacher la section d'encliquetage (14) par actionnement manuel hors de la prise d'encliquetage avec la contre-dépouille et de libérer un mouvement axial relatif des deux sections de couplage (6, 8) l'une par rapport à l'autre au moins dans le sens de traction.
